# EUROPEAN PATENT SPECIFICATION

(11) **EP 1 374 880 B1**
(45) Date of publication and mention of the grant of the patent: **08.03.2006**
(21) Application number: 01274051.0
(22) Date of filing: 01.11.2001
(51) Int. Cl.: A61K 36/00, A61P 35/00

(54) **AN ANTINEOPLASTIC DRUG**
ANTINEOPLASTISCHES ARZNEIMITTEL
MEDICAMENT ANTINEOPLASIQUE

(30) Priority: 02.04.2001 IL 13532501
(43) Date of publication of application: 02.01.2004
(73) Proprietor: Li, Hongfen, Haidian District, Beijing 100000 (CN)
(72) Inventor: Li, Hongfen, Haidian District, Beijing 100000 (CN)
(74) Representative: Goddar, Heinz J.
(86) International application number: PCT/CN2001/001521
(87) International publication number: WO 2002/078722

(56) References cited:
- CN-A- 1 097 621
- CN-A- 1 145 240
- CN-A- 1 157 737
- CN-A- 1 205 880
- US-A- 5 437 866
- US-A- 6 071 521
- CHEN JIN: 'Theory of eliminating the evil factors and restorating healthy energy in traditional Chinese medicine and research on anticancerous Chinese medicinal herb' TRADITIONAL CHINESE MEDICINE OF GUANG XI vol. 8, no. 1, 1985, pages 47 - 49

## Description

### FIELD OF THE INVENTION

The present invention relates to a composition for treatment of tumor. More particularly, the present invention is directed to an antineoplastic composition made from pure Chinese herbal medicine.

### BACKGROUND OF THE INVENTION

According to the data released by the Ministry of Public Health of China, cancer has been ranked as the number one killer in urban areas of China in 1990. More than one million patients die from cancer every year in mainland of China. In the United States, one person dies from cancer every minute, and in addition, there are 3 million patients of cancer, wherein one third of them will eventually die from this disease. Up to date, there are more than 100 kinds of different cancer which have already be found. Although operation, radiotherapy and chemotherapy are used as the main therapeutics, it is well known that the treatment of middle and late stage cancer is very difficulty. There are many patients that have lost the opportunity of operation. Furthermore, chemotherapy and radiotherapy do not always get the expected effect, and moreover, they also have serious side effects, including weakness, anorexia, loss of hair, inhibiting the function of bone marrow hematopoiesis , impairment of liver and kidney and ovary function, etc. After the operation, patients often relapse because the immune system is extremely suppressed for ten hours after operation. It is therefore pertinent that a new therapeutic way is founded to enhance cancer treatment as well as reduce side effects of operation, chemotherapy and radiotherapy. Traditional Chinese Medicine has been used clinically for more than 3000 years with a vast clinical experience. It has played unexpected effects in the treatment of various diseases, including cancer. However, it was not until the last 3 decades that modem scientific methods have been used to test Traditional Chinese Medicine with remarkable achievement.

The research and development of anticancer drug from plants have been very fruitful. And advances in biochemical and pharmacological studies of plants derived drugs have exerted enormous impetus on the research and develop of anticancer drugs from plants. However, the available medicines only emphasized one aspect, for example, only for increasing the function of immune system or only for suppressing tumor or for improving the blood circulation etc.

### SUMMARY OF THE INVENTION

The objective of the present invention is to provide new anti-cancer drugs composed of Traditional Chinese Medicine, which is developed according to the theory of strengthening of body resistance to eliminate pathogenic factors of Traditional Chinese Medicine and the theory of increasing the function of immune system, anti-stress, anti-tumor and improving micro circulation to reverse and destroy tumor cells of Western Medicine. The Clinical trials prove that the new antineoplastic drugs of the present invention possess significant therapeutic efficacy.

### DETAILED DESCRIPTION OF THE INVENTION

The composition for treatment of tumor of the present invention is made from all of the following Chinese herbal medicine: Red Ginseng(PANAX GINSING), Fu Ling(PORIA COCOS), Bai Zhu(ATRACTYLODES MACROCEPHALA), Dang Gui( ANGELIC SINENSIS), Huang Qi( ASTRAGALUS MEMBRANACEUS), Huang Qin(SCUTELLARLA BAICALENSIS), Huang Lian(COPTIS CHINENSIS), Huang Bai(PHELLODRON CHINENSE), Gan CAO(GLYCYRRHIZA URALENSIS), Mai Ya(HORDEUM VULGARE), Bai Hua She She CAO( HEDYOTIS DIFFUSA), Ban Bian Lian(LOBELIA CHINESIS LOUR), Ban Zhi Lian(SCUTELLARIA BARBABA), Shen QU(MASSA FERMENTATA MEDICALIS). From above list of Chinese herbal medicine, the following specific prescriptions can be chosen, in which Prescription 1 is basic one and Prescriptions 2 to 6 are adding or reducing ones.

**PRISCRIPTION 1:**

| Name (pinyin) | Latin Name (species) | % amount (w/w) |
|---|---|---|
| Red Ren Shen | PANAX GINSING | 9.4% |
| Fu Ling | PORIA COCOS | 5.7% |
| Bai Zhu | ATRACTYLODES MACROCEPHALA | 5.7% |
| Dang Gui | ANGELIC SINENSIS | 6.6% |
| Huang Qi | ASTRAGALUS MEMBRANACEUS | 5.7% |
| E zhu | CURCUMA ZEDOARIA | 4.7% |
| Huang QIN | SCUTELLARIA BAICALENSIS | 5.7% |
| Huang Bai | PHELLODRON CHINENSE | 4.7% |
| Huang Lian | COPTIS CHINENSIS | 5.7% |
| Gan Cao | GLYCYRRHIZA URALENSIS | 5.7% |
| Shan Zha | CRATAEGUS PINATIFIDA | 4.7% |
| Mai Ya | HORDEUM VULGARE | 1.8% |
| Dan Shen | SALVIA MILTIORRHIZA | 4.7% |
| Wu Wei Zi | SCHISANDRA CHINENSIS | 5.7% |
| Bai Hua She She Cao | HEDYOTIS DIFFUSA | 6.6% |
| Mai Men Dong | OPHIOPHOGON JAPONICUS | 4.7% |
| Ban Bian Lian | LOBELIA CHINESIS LOUR | 4.7% |
| Ban Zhi Lian | SCUTELLARIA BARBABA | 5.7% |
| SHEN QU | MASSA FERMENTATA MEDICALIS | 1.8% |

**PRESCRIPTION 2:**

| Name (pinyin) | Latin Name (species) | % amount (w/w) |
|---|---|---|
| Red Ren Shen | PANAX GINSING | 9.4(1-15)% |
| Fu Ling | PORIA COCOS | 5.7(1-10)% |
| Bai Zhu | ATRACTYLODES MACROCEPHALA | 5.7(1-10)% |
| Dang Gui | ANGELIC SINENSIS | 6.6(1-10)% |
| Huang Qi | ASTRAGALUS MEMBRANACEUS | 5.7(1-10)% |
| E zhu | CURCUMA ZEDOARIA | 4.7(1-10)% |
| Huang Qin | SCUTELLARIA BAICALENSIS | 5.7(1-25)% |
| Huang Bai | PHELLODRON CHINENSE | 4.7(1-20)% |
| Huang Lian | COPTIS CHINENSIS | 5.7(1-20)% |
| Gan Cao | GLYCYRRHIZA URALENSIS | 5.7(1-15)% |
| Shan Zha | CRATAEGUS PINATIFIDA | 4.7(1-10)% |
| Mai Ya | HORDEUM VULGARE | 1.8(1-20)% |
| Dan Shen | SALVIA MILTIORRHIZA | 4.7(1-15)% |
| Wu Wei Zi | SCHISANDRA CHINENSIS | 5.7(1-15)% |
| Bai Hua She She Cao | HEDYOTIS DIFFUSA | 6.6(1-40)% |
| Mai Men Dong | OPHIOPHOGON JAPONICUS | 4.7(1-20)% |
| Ban Bian Lian | LOBELIA CHINESIS LOUR | 4.7(1-25)% |
| Ban Zhi Lian | SCUTELLARIA BARBABA | 5.7(1-25)% |
| SHEN QU | MASSA FERMENTATA MEDICALIS | 1.8(1-20)% |

**PRESCRIPTION 3:**

| Name (pinyin) | Latin Name (species) | % amount (w/w) |
|---|---|---|
| Red Ren Shen Fu Ling | PANAX GINSING | 9.4(1-30)% |
| | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGALUS MEMBRANACEUS | 5.7(1-30)% |
| E zhu | CURCUMA ZEDOARIA | 4.7(1-30)% |
| Huang QIN | SCUTELLARIA BAICALENSIS | 5.7(1-35)% |
| Huang Bai | PHELLODRON CHINENSE | 4.7(1-30)% |
| Huang Lian | COPTIS CHINENSIS | 5.7(1-30)% |
| Gan Cao | GLYCYRRHIZA URALENSIS | 5.7(1-30)% |
| Shan Zha | CRATAEGUS PINATIFIDA | 4.7(1-30)% |
| Mai Ya | HORDEUM VULGARE | 1.8(1-30)% |
| Dan Shen | SALVIA MILTIORRHIZA | 4.7(1-30)% |
| Wu Wei Zi | SCHISANDRA CHINENSIS | 5.7(1-30)% |
| Bai Hua She She Cao | HEDYOTIS DIFFUSA | 6.6(1-40)% |
| Mai Men Dong | OPHIOPHOGON JAPONICUS | 4.7(1-30)% |
| Ban Bian Lian | LOBELIA CHINESIS LOUR | 4.7(1-35)% |
| Ban Zhi Lian | SCUTELLARIA BARBABA | 5.7(1-35)% |
| SHEN QU | MASSA FERNCENTATA MEDICALIS | 1.8(1-30)% |

**PRESCRIPTION 4:**

| Name (pinyin) | Latin Name (species) | %amount(w/w) |
|---|---|---|
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGELUS MEMBRANACEUS | 5.7(1-30)% |
| Huang QIN | SCUTELLARIA BAICALENSIS | 5.7(1-30)% |
| Huang Lian | COPTIS CHINENSIS | 5.7(1-30)% |
| Huang Bai | PHELLODRON CHINENSE | 4.7(1-30)% |
| Gan Cao | GLYCYRRHIZA URALENSIS | 5.7(1-30)% |
| Mai Ya | HORDEUM VULGARE | 1.8(1-30)% |
| Bai Hua She She Cao | HEDYOTIS DIFFUSA | 6.6(1-40)% |
| Ban Bian Lian | LOBELIA CHINESIS LOUR | 4.7(1-30)% |
| Ban Zhi Lian | SCUTELLARIA BARBABA | 5.7(1-30)% |
| SHEN QU | MASSA FERMENTATA MEDICALIS | 1.8(1-30)% |
| CHAI HU | BUPLEURUM SCORZONERIFOL IUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
| JIANG(GAN or SHENG) | ZINGIBER OFFICINALE(dry or fresh) | 2(1-30)% |

**PRESCRIPTION 5:**

| Name (pinyin) | Latin Name (species) | %amount(w/w) |
|---|---|---|
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SUIENSIS | 6.6(1-30)% |
| Huang Qi | ASTRAGELUS MEMBRANACEUS | 5.7(1-30)% |
| Huang QIN | SCUTELLARIA BAICALENSIS | 5.7(1-30)% |
| Huang Lian | COPTIS CHINENSIS | 5.7(1-30)% |
| Huang Bai | PHELLODRON CHINENSE | 4.7(1-30)% |
| Gan Cao | GLYCYRRHIZA URALENSIS | 5.7(1-30)% |
| Mai Ya | HORDEUM VULGARE | 1.8(1-30)% |
| Bai Hua She She Cao | HEDYOTIS DIFFUSA | 6.6(1-40)% |
| Ban Bian Lian | LOBELIA CHINESIS LOUR | 4.7(1-30)% |
| Ban Zhi Lian | SCUTELLARIA BARBABA | 5.7(1-30)% |
| SHEN QU | MASSA FERMENTATA MEDICALIS | 1.8(1-30)% |
| CHAI HU | BUPLEURUM SCORZONERIFOLIUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |

In the prescription 5, 2 (1-30)% of JIANG (GAN OR SHENG), ZINGIBER OFFICINALE (dry or fresh)) can be added or 4.7(1-30)% of Huang Bai (PHELLODRON CHINENSE) can be cancelled.

**PRESCRIPTION 6:**

| Name (pinyin) | Latin Name (species) | %amount w/w) |
|---|---|---|
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGELUS MEMBRANACEUS | 5.7(1-30)% |
| Huang QIN | SCUTELLARIA BAICALENSIS | 5.7(1-30)% |
| Huang Lian | COPTIS CHINENSIS | 5.7(1-30)% |
| Huang Bai | PHELLODRON CHINENSE | 4.7(1-30)% |
| Gan Cao | GLYCYRRHIZA URALENSIS | 5.7(1-30)% |
| Mai Ya | HORDEUM VULGARE | 1.8(1-30)% |
| Bai Hua She She Cao | HEDYOTIS DIFFUSA | 6.6(1-40)% |
| Ban Bian Lian | LOBELIA CHINESIS LOUR | 4.7(1-30)% |
| Ban Zhi Lian | SCUTELLARIA BARBABA | 5.7(1-30)% |
| SHEN QU | MASSA FERMENTATA MEDICALIS | 1.8(1-30)% |
| CHAI HU | BUPLEURUM SCORZONERIFOLIUM | 5(1-30)% |
| BAN X1A | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
| LING ZHI | GANODERMA LUCIDUM | 6(1-30)% |

In the prescription 6, 2 (1-30)% of JIANG (GAN OR SHENG), ZJNGIBER OFFICINALE (dry or fresh)) can be added, or 4.7(1-30)% of Huang Bai (PHELLODRON CHINENSE) can be cancelled.

Note: In each above-mentioned prescription, the figures shown in parenthesis represent that the percentage amount can vary within the range, for example, Ling Zhi 6(1-30) % in (1-30) means that the percentage amount of Ling Zhi can vary from 1 to 30.

The inventor calls the new antineoplastic drugs of the present invention by the name of Anticancer Number One (hereinafter referred to as simply ACNO). It is a new anticancer drug exacted from traditional Chinese medicines. The prescription is based on Si Junzi Tang (Decoction of Four Noble Drugs) and Huanglian Jiedu Tang (Antidotal Decoction of coptis), and is formed by adding therein drugs for replenishing Qi and blood, and traditional Chinese medicines for detoxifying and anti-tumor. The function of Si Junzi Tang is to replenish Qi and strenghthen the spleen. Among the four ingredients in the prescription, Ginseng is sweet in flavor and warm in nature, possesses the effect of invigorating primordial Qi and acts as a principal drug to strengthen the spleen and nourish the stomach; Bai Zhu having a bitter flavor and warm nature is effective for strengthening the spleen and eliminating dampness and is considered as an assistant drugs; Fu Ling, being sweet and insipid in flavor, exerts the part of an adjuvant drug in achieving the effect of strengthening the spleen; Gan Cao, being sweet in flavor and warm in nature, is used as a guiding drug for regulating the meddle-warmer. In the Huanglian Jiedu Tang, ingredient Huang Lian is used as principal drug, which plays a significant role of purging pathogenic fire in the heart and middle-energizer, Huang Qin acts as an assistant drug with the effect of clearing away heat in the lung and purging fire in the upper-energizer; Huang Bai is used as both adjuvant and guiding drugs, the former provides the effect of purging pathogenic fire in lower- energizer and the latter have the effect of removing pathogenic fire in the tri-energizer by inducing diuresis. In the prescriptions of the present invention, Ginseng, Huang Qi, Bai Zhu, Gan Cao, which are sweet in flavor and warm in nature, are able to strengthen spleen and invigorate the Qi; Dang Gui, which is sweet and pungent in flavor and warm in property, can play the role of tonifying the blood and nourishing the liver; E Zhu relieves the stagnation of Qi, alleviate pain and invigorate blood circulation; Dan Shen can invigorate blood circulation, remove blood stasis, remove heat from blood, treat carbuncles, and tranquilizing the disturbed mind by nourishing the blood; Wu Wei Zi nourish the kidney and tranquilize the mind; Mai Man Dong nourish yin, promote the production of body liquid and clear away heat-fire to relieve vexation, which combines with Ren Shen for treating the injury of Qi and Yin by heat, palpitation and pulse insufficiency of the patient after chemotherapy; Bai hua she she cao, Bai Zhi Lian, and Ban Bian Lian assistant Huang Lian Jie Du Tang with clearing heat and detoxify to fight with tumor; Shan Zha, Mai ya, and Shen Qu strength the spleen and improve digestion in order to prevent the drugs for replenishing Qi and blood (Yi Qi Bu Xue Yao) from growing and loathing Qi to block the spleen and stomach's function of transport and digestion. The action of the whole formula is tonifying but not stagnant, and is purgation but not asthenia. So the formula increases organism's immunity function and inhibits/kills the tumor at the same time. Therefore, the prescriptions of the present invention is developed according to the theory of strengthening of body resistance to eliminate pathogenic factors of Traditional Chinese Medicine and the theory of increasing the function of immune system, anti-stress; anti-tumor and improving micro circulation to reverse and destroy tumor cells of Western Medicine. The inventor has used clinically ACNO for 15 years in china and more than 5 years in Israel to treat thousands patients of cancer with excellent clinical efficacy results. Distinguishing from Western medicine, the main principles of treatment cancer in anticancer number one are: searching for the primary cause of disease in treatment, and treating the physically and mentally as a whole instead of paying attention to the local target lesion only, so this therapeutic method was easy, effective, and safe without adverse effect, which is suitable for elder and asthenics, who are unable or failed to receive operation, chemotherapy and radiotherapy. It has been proved by clinical practice that ACNO inhibits/kills cancer cells (eliminating pathogenic factors); enhance the immune function of the organism, increase NK cells activity; possess the function of antistress and improve micro-circulation and suppress the genesis and metastasis of tumor; increase sensitivity to other therapies and diminish toxic and adverse action of other therapies and drugs. So ACNO enhances the body's anti-tumor capacity, improve the patient's quality of life and reduce or prevent the genesis, relapse and metastasis of tumor. For further proving the therapeutic efficacy, the animal trials were made in Tel-Aviv University of Israel to test the effects of ACNO on natural killer cell activity and metastasis of tumor.

### RESEARCHES IN ANIMALS

### EXPERMENTS MATERALS

1.Fischer -344 male and female rats (Harlan Laboratories of Jerusalem, Israel) were housed four in a cage with free access to food and water ad libitum in a 12:12 hours lighting regiment. Animals were acclimatized to the surrounding for at least 3 weeks before any experiment, and were weighed three times. All experiments were conducted during the first half of the light phase. In each experiment, all animal were of the same age.
2. ACNO was made in China, which contained Red Ginseng, Atractylodes Macrocephala, Poria Cocos,etc.
3.The experimental animals were divided into test group and control group. The animals in the test group were fed with 0.1, 0.5, 2g/kg body weight ACNO mixed and water added standard powdered rodent food (test food) every day. The animals in the control group received the same wet food mixture without ACNO (control food)in the same amount. All animals were fed at regular time every day. To adjust animals to the feeding protocol, all rats received the wet mixture without ACNO for 3 days before each experiment. Thereafter, the animal were fed test food and control food, respectively. Rats from all groups consumed more than 95% of the food each day, approximately 70% of it was consumed during the dark phase. Body weight of each animal was monitored twice a week during the experiment.
4. MADB106 tumor cell: MADB106 cell was obtained from a pulmonary metastasis of a mammary adenocarcinoma (MADB100) by a special incubation.
5. Induction and counting of tumor metastasis
   Rats were lightly anesthetized with halothane, and then 10⁵ MADB106 tumor cells were injected into their tail vein. At the same time, number 3 and 4 of the animals in every group were injected with 0.8ml β-receptor agonist(Metaproterenol MP). Rats were anesthetized with halothane after they were kept on feeding with ACNO for 3 weeks counted from the day of tumor inoculation, and their lungs were removed and placed in Bouin solution (72% saturated picric acid solution, 23% formaldehyde [37%solution] and 5% glacial acetic acid) for 24 h. Thereafter, the lungs were washed with ethanol, and then the cell number of pulmonary metastasis were counted by two experimenters unaware of the group origin (in blind way), independently. The obtained data were analyzed statistically.
6. After the animals had fed with the test food for 2 weeks, 1ml of blood was draw by cardiac puncture to detect the number of NK cells per ml and the activity thereof.

### Statistical analysis

ANOVA method was applied for detecting the number of NK cells per ml and the activity thereof, while T test and ANOVA test were used for the count of the cell number of pulmonary tumor.

### EXPERIMENTAL METHOD

### 1: The effect of ACNO on number and activity of NK cells

78 healthy female Fisher rats with 10 -12 weeks old and 250-350g body weight were randomly divided into 5 groups, wherein 3 groups were administrated by ACNO with high, middle and low dosage (0.1, 0.5, 2.0g/kg body weight/day). There are 14, 15, 14, 15 and 20 rats in low, middle, high dosage groups, Panax ginsing group (0.5g Panax Ginsing /kg body weight/day) and control group (fed with the control food in same amount only), respectively. 2 weeks after adminstration, rats were lightly anesthetized with halothane, blood sample was drawn by cardiac puncture and placed into the tube with heparin, and centrifuged to collect monocytes. The monocytes were washed with PBS twice, and the concentration of the cell were adjusted to 0.25 X 10⁷. Thereafter, the number of NK cells were counted, the activity of NK cells were detected by the method using YAC-1 or MADB106 target cells by releasing ⁵¹Cr or ¹²⁵IDUR.

### 2. The effect of ACNO on susceptibility to tumor cells metastasis

89 healthy female Fischer rats with 7 -8 weeks old and 120-170g body weight (48 females and 61 males) were randomly divided into 2 groups, Experiment group and Control group, wherein There are 40 rats (20 females and 20 males) and 49 rats (20 females and 29 males) in Experiment group and Control group, respectively. The animals in Experiment group were fed with 2g/kg body weight/day ACNO and water added mix food, while those in the Control group were fed with water added mix food in same amount. After 3 weeks of feeding, rats were lightly anesthetized with halothane, and then 0.5ml of 4X105 tumor cells were injected into their tail vein. At the same time, 0.8ml β-receptor agonist (MP) were injected. 3 weeks after inoculating tumor cell, rats were anesthetized with halothane, and then their lungs were removed and fixed in Bouin solution for 24 h. Thereafter, the lungs were washed with ethanol, and then the cell number of pulmonary metastasis were counted by two experimenters unaware of the group origin (in blind way), independently. The obtained data were analyzed statistically.

### RESULTS

The effect of ACNO on NK cells:
Rats from all groups consumed 95% of the food each day, and during the experiment period, gained the body weight of an average of 23.4g/week with no significant difference among various groups.

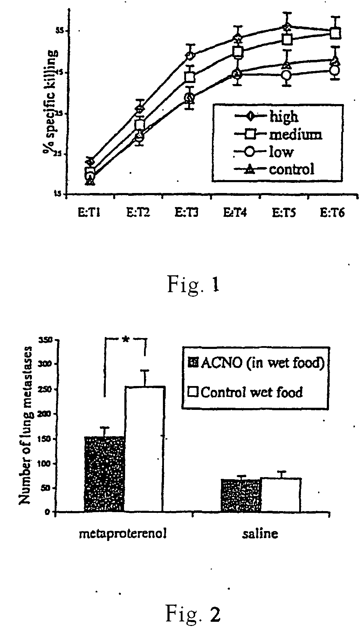

The results showed that ACNO caused a significant dose-dependent increase in activity of NK cells per ml blood, and Fig.1 showed the specific percentages of different E:T rate in various groups. Compared to the animals in the control group, those in the high dose group showed an significant higher activity of NK cells (P<0.0001), and the other administration groups also showed increased activity of NK cells to a different degree without significant difference. This result suggested that the effect of high dose was superior to that of low dose. On the other hand, ACNO also caused an increased number of NK cells/ml blood, wherein the number value of NK cells/ml blood in control group is 344.4±34.9 (mean±SD), that in high dose group is 530±79.1 (mean±SD), that in medium dose group is 407.7±63.6 (mean±SD), that in low dose group is 382.2±53.2 (mean±SD), and that in Panax Ginsing group is 373 ±65.1 (mean±SD). This result demonstrated that ACNO increased significantly the activity and number of NK cells.

### 2. The effect of ACNO on tumor cells metastasis:

During the experimental period, rats from the two groups consumed 95% of the food, and gained an average of 20.5g/week (males) or 10g/week (females), with no significant difference compare to the third group maintained on ad libitum dry food schedule.

With respect to cell number of metastasis, the difference between those counted by the two experimenter was lower than 0.9, and the average thereof was used. The is no significant difference among the three groups (dry food, wet food only, and ACNO group) without the administration of β-receptor agonist. However, compared to the 2 control groups without the administration of β-receptor agonist, the 2 control groups with the administration of β-receptor agonist caused a 4-5.5 folds increase in the cell number of metastases (NS70+14, MP255+32), while ACNO-treated groups only caused a 2.3 folds increase (ACNO + MP154+18, control group + MP255+32), P<0.0006. This experimental result showed that the state of stress and anxiety significantly promoted the tumor metastases, however, ACNO remarkably inhibited the tumor metastases under the state of stress and anxiety.

### THE BENIFICIAL EFFECT OF THE INVENTION

It is considered in Traditional Chinese Medicine that tumorigenesis usually results from external evil invasion, which could cause functional disturbance in Yin and Yang, Qi and blood, channels and collaterals as well as the viscera and bowels. Therefore, the principle of cancer treatment in Traditional Chinese Medicine emphasizes upon regulating visceral function, consolidating the constitution and eliminating pathogenic factors. In recent years, the researchers of western medicine suggest that the main reason of tumorigenesis is that the immune function of patient was inhibited. So increasing immune function of body and inhibiting the cancer cells are very important methods in the treatment of cancer. Natural Killer cell belongs to the immune system of the body. NK cells constitute a fist line of defense against infectious disease and play a very important role in preventing the tumorigenesis and tumor metastases. Animal trials' results obtained in Immunology Lab of Tel-Aviv University of Israel demonstrated convincingly that NK cells played an very important roles in controlling various aspects of tumor development, including the metastatic process, the tumorigenic effects of cancinogenes and the primary seeding of implanted solid tumor. ACNO can increase the number and activity of NK cells and inhibit the growth of tumor and prevent metastasis. It have been suggested that Scutellaria Baicalensis, Coptis Chinesis, and Phellodron Chinensis, which are contained in the ACNO, are cytotoxic anticancer drugs; and Red Ginseng belongs to chemoprophylactic agents of cancer. The resistance of anticancer drugs represents a major cause of failure in cancer chemotherapy, and to resolve this problem, a lot of researches were done and showed red Ginseng is a new reversal agent with non-toxic effects. Also, a series of test was carried out to study the anticancer effects of ginsenoside Rg3, and the pharmacological studys showed (1) antitumor metastasis; (2) anticancer effects: the inhibition rate of Rg3 against B16 melanoma, LEWIS lung cancer, S180 sarcoma by subcutaneous transplantation were 50 - 60%. (3) Immunoenhancement effects: Rg3 administrated by oral route can remarkably enhance the NK and IL-2 activity of lung cancer implated mice and normal mice. (4) Rg3 can enhance the anticancer effect and reduce the side effects of chemotherapy. The studies on mechanism showed that Rg3 can inhibit the proliferation, infiltration and metastasis of tumor cells, and enhance the immunological function of mice bearing tumor. Astragalus membranaceus, salvia miltiorrhiza etc. possess the function of increasing the immunological function of the organisms. The effective components of Curcuma zedoaria is cellular toxic, which inhibits the proliferation of tumor cells by inhibiting the protein synthetic processes of DNA and RNA, which might induce the temination of tumor cells growth by way of blocking the cells respiration in certain stage or destroying the cellular membrane to cause autolysis of tumor cell. Astrragalus Membranaceus combined with chemotherapy, radiotherapy and biological therapy may enhance the therapeutic effect and reduce the side effect thereof. The polysaccharides components of Radix Ginseng has reticuloendothelial system function-enhanced effect and contains trace amounts of Germanium (Ge), which has been known as an effective cancer-preventing element The study of Ge in cancer prevention has vast prospect. The animal trials showed that ACNO can increase cytotoxic activity and number of NK cells and suppress tumor metastasis. This result is consistent with that obtained from clinical trials and therefore is worthy of further research. ACNO, having been applied in Israel for more than 5 years, shall open a new road for preventing and treating cancer.

### BRIEF DESCRIPTION OF THE FIGURES

Figure 1 shows the effects of increasing doses of ACNO (low, medium and high: 0.1, 0.5 and 2g/kg/day) on NK activity (% specific killing) in increasing effector to target (E/T) ratios. ACNO caused a significant dose-dependent increase in NK activity (P<0.0001).
Figure 2 shows the effects of ACNO (2g/kg/day) and of β-receptor agonist (MP) (0.8g/kg body weight) on the cells number of MADB 106 metastases, wherein MP injection significantly increased the cells number of metastases, while ACNO administration significantly decreased the cells number of metastases (P<0.0006).

### THE BEST MODE FOT CARRYING OUT THE INVENTION

The Chinese herbal medicines used in each prescription are available commercially in China. The various medicinal herbs were weighed according to the herbs employed and their percentage showed in the prescription, and then ground to powder to be encapsulated into capsules of 0.5g per capsule.

## Claims

1. A composition for treatment of tumor, comprising all of the following Chinese herbal medicine: Red Ginseng(PANAX GINSING), Fu Ling(PORIA COCOS), Bai Zhu(ATRACTYLODES MACROCEPHALA), Dang Gui(ANGELIC SINENSIS), Huang Qi(ASTRAGALUS MEMBRANACEUS), Huang Qin(SCUTELLARIA BAICALENSIS), Huang Lian(COPTIS CHINESIS), HuangBai(PHELLODRON CHINENSE), Gan CAO(GLYCYRRHIZA URALENSIS), Mai Ya(HORDEUM VULGARE), Bai Hua She She CAO(HEDYOTIS DIFFUSA), Ban Bian Lian(LOBELIA CHINESIS LOUR), Ban Zhi Lian (SCUTELLARIA BARBABA), Shen Qu(MASSA FERMENTATA MEDICALIS).

2. A composition of treatment of tumor according to Claim 1, comprising the following Chinese herbal medicine:
| Name (pinyin) | Latin Name (species) | % amount (w/w) |
|---|---|---|
| Red Ren Shen | PANAX GINSING | 9.4% |
| Fu Ling | PORIA COCOS | 5.7% |
| Bai Zhu | ATRACTYLODES MACROCEPHALA | 5.7% |
| Dang Gui | ANGELIC SINENSIS | 6.6% |
| Huang Qi | ASTRAGALUS MEMBRANACEUS | 5.7% |
| E zhu | CURCUMA ZEDOARIA | 4.7% |
| Huang QIN | SCUTELLARIA BAICALENSIS | 5.7% |
| Huang Bai | PHELLODRON CHINENSE | 4.7% |
| Huang Lian | COPTIS CHINENSIS | 5.7% |
| Gan Cao | GLYCYRRHIZA URALENSIS | 5.7% |
| Shan Zha | CRATAEGUS PINATIFIDA | 4.7% |
| Mai Ya | HORDEUM VILGARE | 1.8% |
| Dan Shen | SALVIA MILTIORRHIZA | 4.7% |
| Wu Wei Zi | SCHISANDRA CHINENSIS | 5.7% |
| Bai Hua She She Cao | HEDYOTIS DIFFUSA | 6.6% |
| Mai Men Dong | OPHIOPHOGON JAPONICUS | 4.7% |
| Ban Bian Lian | LOBELIA CHINESIS LOUR | 4.7% |
| Ban Zhi Lian | SCUTELLARIA BARBABA | 5.7% |
| SHEN QU | MASSA FERMENTATA MEDICALIS | 1.8% |

3. A composition for treatment of tumor according to Claim 1, comprising the following Chinese herbal medicine:
| Name (pinyin) | Latin Name (species) | % amount (w/w), |
|---|---|---|
| | | with the figures in parentheses being range values, and the specific value in front of the parentheses representing a preferred value within the respective range. |
| Red Ren Shen | PANAX GINSING | 9.4(1-15)% |
| Fu Ling | PORIA COCOS | 5.7(1-10)% |
| Bai Zhu | ATRACTYLODES MACROCEPHALA | 5.7(1-10)% |
| Dang Gui | ANGELIC SINENSIS | 6.6(1-10)% |
| Huang Qi | ASTRAGALUS MEMBRANACEUS | 5.7(1-10)% |
| E zhu | CURCUMA ZEDOARIA | 4.7(1-10)% |
| Huang Qin | SCUTELLARIA BAICALENSIS | 5.7(1-25)% |
| Huang Bai | PHELLODRON CHINENSE | 4.7(1-20)% |
| Huang Lian | COPTIS CHINENSIS | 5.7(1-20)% |
| Gan Cao | GLYCYRRHIZA URALENSIS | 5.7(1-15)% |
| Shan Zha | CRATAEGUS PINATIFIDA | 4.7(1-10)% |
| Mai Ya | HORDEUM VULGARE | 1.8(1-20)% |
| Dan Shen | SALVIA MILTIORRHIZA | 4.7(1-15)% |
| Wu Wei Zi | SCHISANDRA CHINENSIS | 5.7(1-15)% |
| Bai Hua She She Cao | HEDYOTIS DIFFUSA | 6.6(1-40)% |
| Mai Men Dong | OPHIOPHOGON JAPONICUS | 4.7(1-20)% |
| Ban Bian Lian | LOBELIA CHINESIS LOUR | 4.7(1-25)% |
| Ban Zhi Lian | SCUTELLARIA BARBABA | 5.7(1-25)% |
| SHEN QU | MASSA FERMENTATA MEDICALIS | 1.8(1-20)% |

4. A composition for treatment of tumor according to Claim 1, comprising the following Chinese herbal medicine:
| | | |
|---|---|---|
| | | with the figures in parentheses being range values, and the specific value in front of the parentheses representing a preferred value within the respective range. |
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGALUS MEMBRANACEUS | 5.7(1-30)% |
| E zhu | CURCUMA ZEDOARIA | 4.7(1-30)% |
| Huang Qin | SCUTELLARIA BAICALENSIS | 5.7(1-35)% |
| Huang Bai | PHELLODRON CHINENSE | 4.7(1-30)% |
| Huang Lian | COPTIS CHINENSIS | 5.7(1-30)% |
| Gan Cao | GLYCYRRHIZA URALENSIS | 5.7(1-30)% |
| Shan Zha | CRATAEGUS PINATIFIDA | 4.7(1-30)% |
| Mai Ya | HORDEUM VULGARE | 1.8(1-30)% |
| Dan Shen | SALVIA MILTIORRHIZA | 4.7(1-30)% |
| Wu Wei Zi | SCHISANDRA CHINENSIS | 5.7(1-30)% |
| Bai Hua She She Cao | HEDYOTIS DIFFUSA | 6.6(1-40)% |
| Mai Men Dong | OPHIOPHOGON JAPONICUS | 4.7(1-30)% |
| Ban Bian Lian | LOBELIA CHINESIS LOUR | 4.7(1-35)% |
| Ban Zhi Lian | SCUTELLARIA BARBABA | 5.7(1-35)% |
| SHEN QU | MASSA FERMENTATA MEDICALIS | 1.8(1-30)% |

5. A composition for treatment of tumor according to Claim 1, comprising the following Chinese herbal medicine:
| | | |
|---|---|---|
| | | with the figures in parentheses being range values, and the specific value in front of the parentheses representing a preferred value within the respective range. |
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGALUS MEMBRANACEUS | 5.7(1-30)% |
| Huang Qin | SCUTELLARIA BAICALENSIS | 5.7(1-30)% |
| Huang Lian | COPTIS CHINENSIS | 5.7(1-30)% |
| Huang Bai | PHELLODRON CHINENSE | 4.7(1-30)% |
| Gan Cao | GLYCYRRHIZA URALENSIS | 5.7(1-30)% |
| Mai Ya | HORDEUM VULGARE | 1.8(1-30)% |
| Bai Hua She She Cao | HEDYOTIS DIFFUSA | 6.6(1-40)% |
| Ban Bian Lian | LOBELIA CHINESIS LOUR | 4.7(1-30)% |
| Ban Zhi Lian | SCUTELLARIA BARBABA | 5.7(1-30)% |
| SHEN QU | MASSA FERMENTATA MEDICALIS | 1.8(1-30)% |
| CHAI HU | BUPLEURUM SCORZONERIFOLIUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
| JIANG(GANorSHENG) | ZINGIBER OFFICINALE (dry or fresh) | 2(1-30)% |

6. A composition for treatment of tumor according to Claim 1, comprising the following Chinese herbal medicine:
| | | |
|---|---|---|
| | | with the figures in parentheses being range values, and the specific value in front of the parentheses representing a preferred value within the respective range. |
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGALUS MEMBRANACEUS | 5.7(1-30)% |
| Huang Qin | SCUTELLARIA BAICALENSIS | 5.7(1-30)% |
| Huang Lian | COPTIS CHINENSIS | 5.7(1-30)% |
| Huang Bai | PHELLODRON CHINENSE | 4.7(1-30)% |
| Gan Cao | GLYCYRRHIZA URALENSIS | 5.7(1-30)% |
| Mai Ya | HORDEUM VULGARE | 1.8(1-30)% |
| Bai Hua She She Cao | HEDYOTIS DIFFUSA | 6.6(1-40)% |
| Ban Bian Lian | LOBELIA CHINESIS LOUR | 4.7(1-30)% |
| Ban Zhi Lian | SCUTELLARIA BARBABA | 5.7(1-30)% |
| SHEN QU | MASSA FERMENTATA MEDICALIS | 1.8(1-30)% |
| CHAI HU | BUPLEURUM SCORZONERIFOLIUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
wherein 2(1-30)% of Jiang (GAN or SHENG), ZINGIBER OFFICINALE (dry or fresh) can be added, or 4.7(1-30)% of Huang Bai (PHELLODRON CHINESE) can be cancelled.

7. A composition for treatment of tumor according to Claim 1, comprising the following Chinese herbal medicine:
| | | |
|---|---|---|
| | | with the figures in parentheses being range values, and the specific value in front of the parentheses representing a preferred value within the respective range. |
| Red Ren Shen | PANAX GINSING | 9.4(1-30)% |
| Fu Ling | PORIA COCOS | 5.7(1-30)% |
| Bai Zhu | ATRACTYLODES MACROCEPHALA | 5.7(1-30)% |
| Dang Gui | ANGELIC SINENSIS | 6.6(1-30)% |
| Huang Qi | ASTRAGALUS MEMBRANACEUS | 5.7(1-30)% |
| Huang Qin | SCUTELLARIA BAICALENSIS | 5.7(1-30)% |
| Huang Lian | COPTIS CHINENSIS | 5.7(1-30)% |
| Huang Bai | PHELLODRON CHINENSE | 4.7(1-30)% |
| Gan Cao | GLYCYRRHIZA URALENSIS | 5.7(1-30)% |
| Mai Ya | HORDEUM VULGARE | 1.8(1-30)% |
| Bai Hua She She Cao | HEDYOTIS DIFFUSA | 6.6(1-40)% |
| Ban Bian Lian | LOBELIA CHINESIS LOUR | 4.7(1-30)% |
| Ban Zhi Lian | SCUTELLARIA BARBABA | 5.7(1-30)% |
| SHEN QU | MASSA FERMENTATA MEDICALIS | 1.8(1-30)% |
| CHAI HU | BUPLEURUM SCORZONERIFOLIUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
| LING ZHI | GANODERMA LUCIDUM | 6(1-30)% |
wherein 2(1-30)% of JIANG (GAN OR SHENG), ZINGIBER OFFICINALE (dry or fresh) can be added, or 4.7(1-30)% of Huang Bai (PHELLODRON CHINENSE) can be cancelled.

## Patentansprüche

1. Zusammensetzung zur Behandlung von Tumoren, umfassend alle der folgenden chinesischen Kräuterarzneien: Roter Ginseng(PANAX GINSING), Fu Ling(PORIA COCOS), Bai Zhu(ATRACTYLODES MACROCEPHALA), Dang Gui(ANGELIC SINENSIS), Huang Qi(ASTRAGALUS MEMBRANACEUS), Huang Qin(SCUTELLARIA BAICALENSIS), Huang Lian(COPTIS CHINENSIS), HuangBai(PHELLODRON CHINENSE), Gan CAO(GLYCYRRHIZA URALENSIS), Mai Ya(HORDEUM VULGARE), Bai Hua She She CAO(HEDYOTIS DIFFUSA), Ban Bian Lian(LOBELIA CHINESIS LOUR), Ban Zhi Lian(SCUTELLARIA BARBABA), Shen Qu(MASSA FERMENTATA MEDICALIS).

2. Zusammensetzung zur Behandlung von Tumoren nach Anspruch 1, umfassend die folgenden chinesischen Kräuterarzneien:
| Name (Pinyin) | Lateinischer Name (Spezies) | % Menge (Gew./Gew.) |
|---|---|---|
| Red Ren Shen | PANAX GINSING | 9,4% |
| Fu Ling | PORIA COCOS | 5,7% |
| Bai Zhu | ATRACTYLODES MACROCEPHALA | 5,7% |
| Dang Gui | ANGELIC SINENSIS | 6,6% |
| Huang Qi | ASTRAGALUS MEMBRANACEUS | 5,7% |
| E zhu | CURCUMA ZEDOARIA | 4,7% |
| Huang QIN | SCUTELLARIA BAICALENSIS | 5,7% |
| Huang Bai | PHELLODRON CHINENSE | 4,7% |
| Huang Lian | COPTIS CHINENSIS | 5,7% |
| Gan Cao | GLYCYRRHIZA URALENSIS | 5,7% |
| Shan Zha | CRATAEGUS PINATIFIDA | 4,7% |
| Mai Ya | HORDEUM VULGARE | 1,8% |
| Dan Shen | SALVIA MILTIORRHIZA | 4,7% |
| Wu Wei Zi | SCHISANDRA CHINENSIS | 5,7% |
| Bai Hua She She Cao | HEDYOTIS DIFFUSA | 6,6% |
| Mai Men Dong | OPHIOPHOGON JAPONICUS | 4,7% |
| Ban Bian Lian | LOBELIA CHINESIS LOUR | 4,7% |
| Ban Zhi Lian | SCUTELLARIA BARBABA | 5,7% |
| SHEN QU | MASSA FERMENTATA MEDICALIS | 1,8% |

3. Zusammensetzung zur Behandlung von Tumoren nach Anspruch 1, umfassend die folgenden chinesischen pflanzlichen Arzneien:
| Name (Pinyin) | Lateinischer Name (Spezies) | % Menge (Gew./Gew.) |
|---|---|---|
| | | Wobei die Zahlen in Klammern Bereichswerte sind, und der spezifische Wert vor den Klammern ein bevorzugten Wert innerhalb des jeweiligen Bereichs darstellt. |
| Red Ren Shen | PANAX GINSING | 9,4(1-15)% |
| Fu Ling | PORIA COCOS | 5,7(1-10)% |
| Bai Zhu | ATRACTYLODES MACROCEPHALA | 5,7(1-10)% |
| Dang Gui | ANGELIC SINENSIS | 6,6(1-10)% |
| Huang Qi | ASTRAGALUS MEMBRANACEUS | 5,7(1-10)% |
| E zhu | CURCUMA ZEDOARIA | 4,7(1-10)% |
| Huang QIN | SCUTELLARIA BAICALENSIS | 5,7(1-25)% |
| Huang Bai | PHELLODRON CHINENSE | 4,7(1-20)% |
| Huang Lian | COPTIS CHINENSIS | 5,7(1-20)% |
| Gan Cao | GLYCYRRHIZA URALENSIS | 5,7(1-15)% |
| Shan Zha | CRATAEGUS PINATIFIDA | 4,7(1-10)% |
| Mai Ya | HORDEUM VULGARE | 1,8(1-20)% |
| Dan Shen | SALVIA MILTIORRHIZA | 4,7(1-15)% |
| Wu Wei Zi | SCHISANDRA CHINENSIS | 5,7(1-15)% |
| Bai Hua She She Cao | HEDYOTIS DIFFUSA | 6,6(1-40)% |
| Mai Men Dong | OPHIOPHOGON JAPONICUS | 4,7(1-20)% |
| Ban Bian Lian | LOBELIA CHINESIS LOUR | 4,7(1-25)% |
| Ban Zhi Lian | SCUTELLARIA BARBABA | 5,7(1-25)% |
| SHEN QU | MASSA FERMENTATA MEDICALIS | 1,8(1-20)% |

4. Zusammensetzung zur Behandlung von Tumoren nach Anspruch 1, umfassend die folgenden chinesischen Kräuterarzneien:
| | | |
|---|---|---|
| | | Wobei die Zahlen in Klammern Bereichswerte sind, und der spezifische Wert vor den Klammern ein bevorzugten Wert innerhalb des jeweiligen Bereichs darstellt. |
| Red Ren Shen | PANAX GINSING | 9,4(1-30)% |
| Fu Ling | PORIA COCOS | 5,7(1-30)% |
| Bai Zhu | ATRACTYLODES MACROCEPHALA | 5,7(1-30)% |
| Dang Gui | ANGELIC SINENSIS | 6,6(1-30)% |
| Huang Qi | ASTRAGALUS MEMBRANACEUS | 5,7(1-30)% |
| E zhu | CURCUMA ZEDOARIA | 4,7(1-30)% |
| Huang QIN | SCUTELLARIA BAICALENSIS | 5,7(1-35)% |
| Huang Bai | PHELLODRON CHINENSE | 4,7(1-30)% |
| Huang Lian | COPTIS CHINENSIS | 5,7(1-30)% |
| Gan Cao | GLYCYRRHIZA URALENSIS | 5,7(1-30)% |
| Shan Zha | CRATAEGUS PINATIFIDA | 4,7(1-30)% |
| Mai Ya | HORDEUM VULGARE | 1,8(1-30)% |
| Dan Shen | SALVIA MILTIORRHIZA | 4,7(1-30)% |
| Wu Wei Zi | SCHISANDRA CHINENSIS | 5,7(1-30)% |
| Bai Hua She She Cao | HEDYOTIS DIFFUSA | 6,6(1-40)% |
| Mai Men Dong | OPHIOPHOGON JAPONICUS | 4,7(1-30)% |
| Ban Bian Lian | LOBELIA CHINESIS LOUR | 4,7(1-35)% |
| Ban Zhi Lian | SCUTELLARIA BARBABA | 5,7(1-35)% |
| SHEN QU | MASSA FERMENTATA MEDICALIS | 1,8(1-30)% |

5. Zusammensetzung zur Behandlung von Tumoren nach Anspruch 1, umfassend die folgenden chinesischen Kräuterarzneien:
| | | |
|---|---|---|
| | | Wobei die Zahlen in Klammern Bereichswerte sind, und der spezifische Wert vor den Klammern ein bevorzugten Wert innerhalb des jeweiligen Bereichs darstellt. |
| Red Ren Shen | PANAX GINSING | 9,4(1-30)% |
| Fu Ling | PORIA COCOS | 5,7(1-30)% |
| Bai Zhu | ATRACTYLODES | 5,7(1-30)% |
| | MACROCEPHALA | |
| Dang Gui | ANGELIC SINENSIS | 6,6(1-30)% |
| Huang Qi | ASTRAGALUS MEMBRANACEUS | 5,7(1-30)% |
| Huang QIN | SCUTELLARIA BAICALENSIS | 5,7(1-30)% |
| Huang Lian | COPTIS CHINENSIS | 5,7(1-30)% |
| Huang Bai | PHELLODRON CHINENSE | 4,7(1-30)% |
| Gan Cao | GLYCYRRHIZA URALENSIS | 5,7(1-30)% |
| Mai Ya | HORDEUM VULGARE | 1,8(1-30)% |
| Bai Hua She She Cao | HEDYOTIS DIFFUSA | 6,6(1-40)% |
| Ban Bian Lian | LOBELIA CHINESIS LOUR | 4,7(1-30)% |
| Ban Zhi Lian | SCUTELLARIA BARBABA | 5,7(1-30)% |
| SHEN QU | MASSA FERMENTATA MEDICALIS | 1,8(1-30)% |
| CHAI HU | BUPLEURUM SCORZONERIFOLIUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
| JIANG(GAN oder SHENG) | ZINGIBER OFFICINALE | |
| | (trocken oder frisch) | 2(1-30)% |

6. Zusammensetzung zur Behandlung von Tumoren nach Anspruch 1, umfassend die folgenden chinesischen Kräuterarzneien:
| | | |
|---|---|---|
| | | Wobei die Zahlen in Klammern Bereichswerte sind, und der spezifische Wert vor den Klammern ein bevorzugten Wert innerhalb des jeweiligen Bereichs darstellt. |
| Red Ren Shen | PANAX GINSING | 9,4(1-30)% |
| Fu Ling | PORIA COCOS | 5,7(1-30)% |
| Bai Zhu | ATRACTYLODES | 5,7(1-30)% |
| | MACROCEPHALA | |
| Dang Gui | ANGELIC SINENSIS | 6,6(1-30)% |
| Huang Qi | ASTRAGALUS MEMBRANACEUS | 5,7(1-30)% |
| Huang QIN | SCUTELLARIA BAICALENSIS | 5,7(1-30)% |
| Huang Lian | COPTIS CHINENSIS | 5,7(1-30)% |
| Huang Bai | PHELLODRON CHINENSE | 4,7(1-30)% |
| Gan Cao | GLYCYRRHIZA URALENSIS | 5,7(1-30)% |
| Mai Ya | HORDEUM VULGARE | 1,8(1-30)% |
| Bai Hua She She Cao | HEDYOTIS DIFFUSA | 6,6(1-40)% |
| Ban Bian Lian | LOBELIA CHINESIS LOUR | 4,7(1-30)% |
| Ban Zhi Lian | SCUTELLARIA BARBABA | 5,7(1-30)% |
| SHEN QU | MASSA FERMENTATA MEDICALIS | 1,8(1-30)% |
| CHAI HU | BUPLEURUM SCORZONERIFOLIUM | 5(1-30)% |
| BAN XIA | FINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
wobei 2(1-30)% von Jiang (GAN oder SHENG), ZINGIBER OFFICINALE (trocken oder frisch) zugegeben werden kann, oder 4,7(1-30)% an Huang Bai (PHELLODRON CHINENSE) weggelassen werden können.

7. Zusammensetzung zur Behandlung von Tumoren nach Anspruch 1, umfassend die folgenden chinesischen Kräuterarzneien:
| | | |
|---|---|---|
| | Wobei die Zahlen in Klammern Bereichswerte sind, und der spezifische Wert vor den Klammern ein bevorzugten Wert innerhalb des jeweiligen Bereichs darstellt. | |
| Red Ren Shen | PANAX GINSING | 9,4(1-30)% |
| Fu Ling | PORIA COCOS | 5,7(1-30)% |
| Bai Zhu | ATRACTYLODES | 5,7(1-30)% |
| | MACROCEPHALA | |
| Dang Gui | ANGELIC SINENSIS | 6,6(1-30)% |
| Huang Qi | ASTRAGALUS MEMBRANACEUS | 5,7(1-30)% |
| Huang QIN | SCUTELLARIA BAICALENSIS | 5,7(1-30)% |
| Huang Lian | COPTIS CHINENSIS | 5,7(1-30)% |
| Huang Bai | PHELLODRON CHINENSE | 4,7(1-30)% |
| Gan Cao | GLYCYRRHIZA URALENSIS | 5,7(1-30)% |
| Mai Ya | HORDEUM VULGARE | 1,8(1-30)% |
| Bai Hua She She Cao | HEDYOTIS DIFFUSA | 6,6(1-40)% |
| Ban Bian Lian | LOBELIA CHINESIS LOUR | 4,7(1-30)% |
| Ban Zhi Lian | SCUTELLARIA BARBABA | 5,7(1-30)% |
| SHEN QU | MASSA FERMENTATA MEDICALIS | 1,8(1-30)% |
| CHAI HU | BUPLEURUM SCORZONERIFOLIUM | 5(1-30)% |
| BAN XIA | PINELLIA TERNATA | 5(1-30)% |
| CHEN PI | CITRUS RETICULATA | 5(1-30)% |
| CHUAN LIAN ZI | MELIA TOOSENDAN | 5(1-30)% |
| BAI SHAO | PAEONIA LACTIFLORA | 4(1-30)% |
| LING ZHI | GANODERMA LUCIDUM | 6(1-30)% |
wobei 2(1-30)% an JIANG (GAN ODER SHENG), ZINGIBER OFFICINALE (trocken oder frisch) zugegeben werden können, oder 4,7(1-30)% an Huang Bai (PHELLODRON CHINENSE) weggelassen werden können.

## Revendications

1. Composition pour le traitement de tumeur comprenant la totalité des herbes médicinales chinoises suivantes : Red Ginseng (PANAX GINSING), Fu Ling (PORIA COCOS), Bai Zhu(ATRACTYLODES MACROCEPHALA), Dang Gui(ANGELIC SINENSIS), Huang Qi(ASTRAGALUS MEMBRANACEUS), Huang Qin(SCUTELLARIA BAICALENSIS), Huang Lian(COPTIS CHINESIS), HuangBai(PHELLODRON CHINENSE), Gan CAO(GLYCYRRHIZA URALENSIS), Mai Ya(HORDEUM VULGARE), Bai Hua She She CAO(HEDYOTIS DIFFUSA), Ban Bian Lian(LOBELIA CHINESIS LOUR), Ban Zhi Lian(SCUTELLARIA BARBABA), Shen Qu(MASSA FERMENTATA MEDICALIS).

2. Composition de traitement de tumeur selon la revendication 1, comprenant les herbes médicinales chinoises suivantes :
| Nom (pinyine) | Nom latin (espèces) | Quantité en % (p/p) |
|---|---|---|
| Red Ren Shen | PANAX GINSING | 9,4 % |
| Fu Ling | PORIA COCOS | 5,7 % |
| Bai Zhu | ATRACTYLODES MACROCEPHALA | 5,7 % |
| Dang Gui | ANGELIC SINENSIS | 6,6 % |
| Huang Oi | ASTRAGALUS MEMBRANACEUS | 5,7 % |
| E zhu | CURCUMA ZEDOARIA | 4,7 % |
| Huang QIN | SCUTELLARIA BAICALENSIS | 5,7 % |
| Huang Bai | PHELLODRON CHINENSE | 4,7 % |
| Huang Lian | COPTIS CHINENSIS | 5,7 % |
| Gan Cao | GLYCYRRHIZA URALENSIS | 5,7 % |
| Shan Zha | CRATAEGUS PINATIFIDA | 4,7 % |
| Mai Ya | HORDEUM VILGARE | 1,8 % |
| Dan Shen | SALVIA MILTIORRHIZA | 4,7 % |
| Wu Wei Zi | SCHISANDRA CHINENSIS | 5,7 % |
| Bai Hua She She Cao | HEDYOTIS DIFFUSA | 6,6 % |
| Mai Men Dong | OPHIOPHOGON JAPONICUS | 4,7 % |
| Ban Bian Lian | LOBELIA CHINESIS LOUR | 4,7 % |
| Ban Zhi Lian | SCUTELLARIA BARBABA | 5,7 % |
| SHEN QU | MASSA FERMENTATA | 1,8 % |
| | MEDICALIS | |

3. Composition pour le traitement de tumeur selon la revendication 1, comprenant les herbes médicinales chinoises suivantes :
| | | |
|---|---|---|
| Nom (pinyine) | Nom latin (espèces) | Quantité en % (p/p), avec les chiffres entre parenthèses étant des valeurs de plage et la valeur spécifique à l'avant des parenthèses représentant une valeur préférée à l'intérieur de la plage respective. |
| Red Ren Shen | PANAX GINSING | 9,4(1 à 15)% |
| Fu Ling | PORIA COCOS | 5,7(1 à 10)% |
| Bai Zhu | ATRACTYLODES MACROCEPHALA | 5,7(1 à 10)% |
| Dang Gui | ANGELIC SINENSIS | 6,6(1 à 10)% |
| Huang Qi | ASTRAGALUS MEMBRANACEUS | 5,7(1 à 10)% |
| E zhu | CURCUMA ZEDOARIA | 4,7(1 à 10)% |
| Huang Qin | SCUTELLARIA BAICALENSIS | 5,7(1 à 25)% |
| Huang Bai | PHELLODRON CHINENSE | 4,7(1 à 20)% |
| Huang Lian | COPTIS CHINENSIS | 5,7(1 à 20)% |
| Gan Cao | GLYCYRRHIZA URALENSIS | 5,7(1 à 15)% |
| Shan Zha | CRATAEGUS PINATIFIDA | 4,7(1 à 10)% |
| Mai Ya | HORDEUM VULGARE | 1,8(1 à 20)% |
| Dan Shen | SALVIA MILTIORRHIZA | 4,7(1 à 15)% |
| Wu Wei Zi | SCHISANDRA CHINENSIS | 5,7(1 à 15)% |
| Bai Hua She She Cao | HEDYOTIS DIFFUSA | 6,6(1 à 40)% |
| Mai Men Dong | OPHIOPHOGON JAPONICUS | 4,7(1 à 20)% |
| Ban Bian Lian | LOBELIA CHINESIS LOUR | 4,7(1 à 25)% |
| Ban Zhi Lian | SCUTELLARIA BARBABA | 5,7(1 à 25)% |
| SHEN QU | MASSA FERMENTATA MEDICALIS | 1,8(1 à 20)% |

4. Composition pour le traitement de tumeur selon la revendication 1, comprenant les herbes médicinales chinoises suivantes :
| | | |
|---|---|---|
| | | avec les chiffres entre parenthèses étant des valeurs de plage et la valeur spécifique à l'avant des parenthèses représentant une valeur préférée à l'intérieur de la plage respective. |
| Red Ren Shen | PANAX GINSING | 9,4(1 à 30)% |
| Fu Ling | PORIA COCOS | 5,7(1 à 30)% |
| Bai Zhu | ATRACTYLODES MACROCEPHALA | 5,7(1 à 30)% |
| Dang Gui | ANGRLIC SINENSIS | 6,6(1 à 30)% |
| Huang Qi | ASTRAGALUS | 5,7(1 à 30)% |
| | MEMBRANACEUS | |
| E zhu | CURCUMA ZEDOARIA | 4,7(1 à 30)% |
| Huang Qin | SCUTELLARIA BAICALENSIS | 5,7(1 à 35)% |
| Huang Bai | PHELLODRON CHINENSE | 4,7(1 à 30)% |
| Huang Lian | COPTIS CHINENSIS | 5,7(1 à 30)% |
| Gan Cao | GLYCYRRHIZA URALENSIS | 5,7(1 à 30)% |
| Shan Zha | CRATAEGUS PINATIFIDA | 4,7(1 à 30)% |
| Mai Ya | HORDEUM VULGARE | 1,8(1 à 30)% |
| Dan Shen | SALVIA MILTIORRHIZA | 4,7(1 à 30)% |
| Wu Wei Zi | SCHISANDRA CHINENSIS | 5,7(1 à 30)% |
| Bai Hua She She Cao | HEDYOTIS DIFFUSA | 6,6(1 à 40)% |
| Mai Men Dong | OPHIOPHOGON JAPONICUS | 4,7(1 à 30)% |
| Ban Bian Lian | LOBELIA CHINESIS LOUR | 4,7(1 à 35)% |
| Ban Zhi Lian | SCUTELLARIA BARBARA | 5,7(1 à 35)% |
| SHEN QU | MASSA FERMENTATA MEDICALIS | 1,8(1 à 30)% |

5. Composition pour le traitement de tumeur selon la revendication 1, comprenant les herbes médicinales chinoises suivantes :
| | | |
|---|---|---|
| | | avec les chiffres entre parenthèses étant des valeurs de plage et la valeur spécifique à l'avant des parenthèses représentant une valeur préférée à l'intérieur de la plage respective. |
| Red Ren Shen | PANAX GINSING | 9,4(1 à 30)% |
| Fu Ling | PORIA COCOS | 5,7(1 à 30)% |
| Bai Zhu | ATRACTYLODES | 5,7(1 à 30)% |
| | MACROCEPHALA | |
| Dang Gui | ANGELIC SINENSIS | 6,6(1 à 30)% |
| Huang Qi | ASTRAGALUS | 5,7(1 à 30)% |
| | MEMBRANACEUS | |
| Huang Qin | SCUTELLARIA | 5,7(1 à 30)% |
| | BAICALENSIS | |
| Huang Lian | COPTIS CHINENSIS | 5,7(1 à 30)% |
| Huang Bai | PHELLODRON CHINENSE | 4,7(1 à 30)% |
| Gan Cao | GLYCYRRHIZA | 5,7(1 à 30)% |
| | URALENSIS | |
| Mai Ya | HORDEUM VULGARE | 1,8(1 à 30)% |
| Bai Hua She She Cao | HEDYOTIS DIFFUSA | 6,6(1 à 40)% |
| Ban Bian Lian | LOBELIA CHINESIS | 4,7(1 à 30)% |
| | LOUR | |
| Ban Zhi Lian | SCUTELLARIA BARBABA | 5,7(1 à 30)% |
| SHEN QU | MASSA FERMENTATA | 1,8(1 à 30)% |
| | MEDICALIS | |
| CHAI HU | BUPLEURUM | 5(1 à 30)% |
| | SCORZONERIFOLIUM | |
| BAN XIA | PINELLIA TERNATA | 5(1 à 30)% |
| CHEN PI | CITRUS RETICULATA | 5(1 à 30)% |
| CHUAN LIAN ZI | MELIA TOOSENDAN | 5(1 à 30)% |
| BAI SHAO | PAEONIA LACTIFLORA | 4(1 à 30)% |
| JIANG (GAN ou | ZINGIBER OFFICINALE | 2(1 à 30)% |
| SHENG) | (sèche ou nouvelle) | |

6. Composition pour le traitement de tumeur selon la revendication 1, comprenant les herbes médicinales chinoises suivantes :
| | | |
|---|---|---|
| | | avec les chiffres entre parenthèses étant des valeurs de plage et la valeur spécifique à l'avant des parenthèses représentant une valeur préférée à l'intérieur de la plage respective. |
| Red Ren Shen | PANAX GINSING | 9,4(1 à 30)% |
| Fu Ling | PORIA COCOS | 5,7(1 à 30)% |
| Bai Zhu | ATRACTYLODES MACROCEPHALA | 5,7(1 à 30)% |
| Dang Gui | ANGELIC SINENSIS | 6,6(1 à 30)% |
| Huang Qi | ASTRAGALUS MEMBRANACEUS | 5,7(1 à 30)% |
| Huang Qin | SCUTELLARIA BAICALENSIS | 5,7(1 à 30)% |
| Huang Lian | COPTIS CHINENSIS | 5,7(1 à 30)% |
| Huang Bai | PHELLODRON CHINENSE | 4,7(1 à 30)% |
| Gan Cao | GLYCYRRHIZA URALENSIS | 5,7(1 à 30)% |
| Mai Ya | HORDEUM VULGARE | 1,8(1 à 30) % |
| Bai Hua She Cao | She HEDYOTIS DIFFUSA | 6,6(1 à 40) % |
| Ban Bian Lian | LOBELIA CHINESIS LOUR | 4,7(1 à 30)% |
| Ban Zhi Lian | SCUTELLARIA BARBABA | 5,7(1 à 30)% |
| SHEN QU | MASSA FERMENTATA MEDICALIS | 1,8(1 à 30)% |
| CHAI HU | BUPLEURUM SCORZONERIFOLIUM | 5(1 à 30)% |
| BAN XIA | PINELLIA TERNATA | 5(1 à 30)% |
| CHEN PI | CITRUS RETICULATA | 5(1 à 30)% |
| CHUAN LIAN ZI | MELIA TOOSENDAN | 5(1 à 30) % |
| BAI SHAO | PAEONIA LACTIFLORA | 4(1 à 30)% |
dans laquelle 2 (1 à 30)% de Jiang (GAN ou SHENG), ZINGIBER OFFICINALE (sèche ou nouvelle) peut être ajouté ou 4,7(1 à 30)% de Huang Bai (PHELLODRON CHINESE) peut être annulé.

7. Composition pour le traitement de tumeur selon la revendication 1, comprenant les herbes médicinales chinoises suivantes :
| | | |
|---|---|---|
| | | avec les chiffres entre parenthèses étant des valeurs de plage et la valeur spécifique à l'avant des parenthèses représentant une valeur préférée à l'intérieur de la plage respective. |
| Red Ren Shen | PANAX GINSING | 9,4(1 à 30)% |
| Fu Ling | PORIA COCOS | 5,7(1 à 30)% |
| Bai Zhu | ATRACTYLODES MACROCEPHALA | 5,7(1 à 30)% |
| Dang Gui | ANGELIC SINENSIS | 6,6(1 à 30)% |
| Huang Qi | ASTRAGALUS MEMBRANACEUS | 5,7(1 à 30)% |
| Huang Qin | SCUTELLARIA HAICALENSIS | 5,7(1 à 30)% |
| Huang Lian | COPTIS CHINENSIS | 5,7(1 à 30)% |
| Huang Bai | PHELLODRON CHINENSE | 4,7(1 à 30)% |
| Gan Cao | GLYCYRRHIZA URALENSIS | 5,7(1 à 30)% |
| Mai Ya | HORDEUM VULGARE | 1,8(1 à 30)% |
| Bai Hua She Cao | She HEDYOTIS DIFFUSA | 6,6(1 à 40)% |
| Ban Bian Lian | LOBELIA CHINESIS LOUR | 4,7(1 à 30)% |
| Ban Zhi Lian | SCUTELLARIA BARBABA | 5,7(1 à 30)% |
| SHEN QU | MASSA FERMENTATA MEDICALIS | 1,8(1 à 30)% |
| CHAI HU | BUPLEURUM SCORZONERIFOLIUM | 5(1 à 30)% |
| BAN XIA | PINELLIA TERNATA | 5(1 à 30)% |
| CHEN PI | CITRUS RETICULATA | 5(1 à 30)% |
| CHUAN LIAN ZI | MELIA TOOSENDAN | 5(1 à 30)% |
| BAI SHAO | PAEONIA LACTIFLORA | 4(1 à 30)% |
| LING ZHI | GANODERMA LUCIDUM | 6(1 à 30)% |
dans laquelle 2(1 à 30)% de JIANG (GAN ou SHENG), ZINGIBER OFFICINALE (sèche ou nouvelle) peut être ajouté ou 4,7(1 à 30)% de Huang Bal (PHELLODRON CHINESE) peut être annulé.
